# EUROPEAN PATENT APPLICATION

(11) **EP 2 965 624 A1**
(43) Date of publication of application: **13.01.2016**
(21) Application number: 15176497.4
(22) Date of filing: 13.07.2015
(51) Int. Cl.: A01N 25/30, A01N 59/00, A01N 37/36, A61L 2/18, A01P 1/00

(54) **CONCENTRATE FOR A DISINFECTANT, DISINFECTANT AND PROCESS FOR DISINFECTING A SURFACE**

(30) Priority: 11.07.2014 NL 2013176
(71) Applicant: TheOxide Holding B.V., 5644 KS Eindhoven (NL)
(72) Inventor: Verhaar, Henk, 1343 AJ Almere (NL)
(74) Representative: Patentwerk B.V.

(57) **Abstract**

The present invention relates to a concentrate for a disinfectant, comprising hydrogen peroxide, at least one carboxylic acid and peroxy acid thereof, said at least one carboxylic acid is selected from the group consisting of alpha hydroxy acids and at least one surfactant selected from the group consisting of ionic surfactants, wherein the weight ratio of carboxylic acid and peroxy acid to surfactant is about 50 : 1 to about 5000 : 1, preferably about 50 : 1. The present invention further relates to a disinfectant comprising the concentrate of the present invention as well as a process for disinfecting a surface to be disinfected and the use of a hydrotropic surfactant in a concentrate for a disinfectant.

## Description

The present invention relates to a concentrate for a disinfectant as well as to a disinfectant comprising the concentrate of the present invention. The present invention further relates to a process for disinfecting a surface and the use of a surfactant in a concentrate for a disinfectant.

Disinfecting compositions, e.g. antimicrobial surface sanitizing compositions, are well known in the art. For example US patent 5,139,788 discloses an antimicrobial surface sanitizing composition comprising a major portion of diluent and an active antimicrobial agent, wherein the agent comprises an antimicrobial effective amount of a hydroxy substituted carboxylic acid and an antimicrobial effective amount of hydrogen peroxide.

International patent application WO 97/28691 A1 discloses a low odor, aqueous, quick acting room temperature disinfectant solution primarily useful for medical instruments to disinfect within a half hour or less. The composition comprises a reacting or synergistic combination of hydrogen peroxide and about 1% to 30% by weight of a water soluble organic acid or salt form thereof with the acid preferably being selected from the group consisting of malonic and succinic acids.

United States Patent US 6,168,808 B1 discloses a composition in the form of an aqueous solution, useful for disinfecting hard surfaces comprising peracetic acid, acetic acid, hydrogen peroxide, amine oxide and, optionally, a non-ionic surfactant.

International patent application WO 03/067989 A1 discloses an enhanced activity aqueous disinfecting solution having a pH of from about 0.5 to about 6 and consisting essentially of hydrogen peroxide, at least one anionic surfactant. Optionally, the solution may contain at least one additional ingredient chosen from a monocarboxylic acid, a polycarboxylic acid, and mixtures thereof, and at least one further additional ingredient chosen from benzyl alcohol, an alcohol.

International patent application WO 2005/014057 A1 discloses a composition, comprising: greater than about 0.1% by weight hydrogen peroxide, an aromatic acid component, surfactant, optionally, a solvent and a carrier. The composition disclosed is useful as a disinfecting composition for killing microorganisms such as bacterium (including Mycobacterium), spores and fungi.

The present invention provides an improved concentrate for a disinfectant. The invention provides hereto a concentrate for a disinfectant, comprising hydrogen peroxide, at least one carboxylic acid and peroxy acid thereof, said at least one carboxylic acid is selected from the group consisting of alpha hydroxy acids and at least one surfactant selected from the group consisting of ionic surfactants. The concentrate of the present invention is further characterized in that the weight ratio of carboxylic acid and peroxy acid to surfactant is about 50 : 1 to about 5000 : 1, preferably about 50 : 1. The weight ratio of hydrogen peroxide to carboxylic acid and peroxy acid is, preferably, about 1 : 1.

It was found that by using an ionic surfactant in a concentrate using a significant low amount of the surfactant, i.e. within the range of the above defined weight ratio, an increase in disinfecting efficacy was observed compared to the disinfecting compositions known in the art. It was observed that a more than 5 log reduction of bacteria, e.g. *Staphylococcus aureus, Pseudomonas aeruginosa, Escherichia coli* and *Enterococcus hirae,* could be reached within 60 seconds with unusually low concentrations of disinfectant. Also a more than 4 log reduction of yeasts, e.g. *Candida albicans,* could be reached within 60 seconds as well. Such an increase in disinfecting efficacy at low amounts of surfactant used in the composition was not expected, since the disinfecting efficacy would be expected to decrease with a decrease in amount of surfactant used. The present invention therefore provides the insight that still a synergistic effect is observed by using low amounts of an ionic surfactant in combination with a peroxy acid of an alpha hydroxy acid.

The at least one surfactant used in the concentrate of the present invention may comprise an anionic surfactant including a sulfosuccinate, such as an alkyl sulfosuccinate, in particular a dialkyl sulfosuccinate. A particular increase in disinfecting efficacy is observed wherein the at least one surfactant comprises an ammonium dinonyl sulfosuccinate, diamyl sodium sulfosuccinate, dicapryl sodium sulfosuccinate, diheptyl sodium sulfosuccinate, dihexyl sodium sulfosuccinate, diisobutyl sodium sulfosuccinate, ditridecyl sodium sulfosuccinate and/or dioctyl sodium sulfosuccinate. The use of dioctyl sodium solfosuccinate as a surfactant is in particular preferred.

The amount of the at least one carboxylic acid in combination with the amount of the hydrogen peroxide comprised in the concentrate of the present invention is chosen such that the concentrate further comprises at least one peroxy acid of the at least one carboxylic acid. The carboxylic acid of the present invention may be selected from lactic acid, citric acid, glycolic acid, 2-hydroxybutyric acid and combinations thereof. The use of lactic acid is in particular preferred obtaining good disinfecting results.

In a preferred embodiment, the concentrate of the present invention is soluble in a liquid medium, such as an aqueous medium, e.g. water. Preferably, the concentrate of the present invention is in the form of an aqueous solution.

In a further embodiment of the present invention the concentrate may comprise:
- 0.25 - 25 weight-% hydrogen peroxide;
- 0.25 - 25 weight-% carboxylic acid and peroxy acid thereof; and
- 0.005 - 0.5 weight-% surfactant,
wherein the weight percentages are calculated based on the total weight of the concentrate. It is further noted that the concentrate of the present invention may further comprise water. Even further, the pH of the concentrate of the present invention is preferably in the range of 2 to 5.

In an embodiment of the present invention, the concentrate may comprise up to about 1.5 weight-% peroxy acid, wherein the weight percentage is calculated based on the total weight of the concentrate. Preferably, the amount of peroxy acid is in the range of 0.01 to 1.5 weight-% of the total weight of the concentrate

In another aspect of the present invention, the present invention relates to a disinfectant comprising the above-described concentrate wherein the concentrate is solubilized in a liquid medium, such as an aqueous medium, e.g. water. Consequently, the disinfectant is preferably in the range of 2 to 5.

It was found that the disinfectant may still be effective, i.e. at least a 5 log reduction of bacteria and/or at least a 4 log reduction of yeasts, in case the disinfectant comprises at least 1 part concentrate dissolved in 100 parts aqueous medium, e.g. water. It has to be understood that more than 1 part concentrate may be dissolved in 100 parts aqueous medium. Preferably, the amount of hydrogen peroxide and/or carboxylic acid is in the range of 0.25 to 2.5 weight-% of the total weight of the disinfectant.

Furthermore, effective disinfectant properties, i.e. at least a 5 log reduction of bacteria and/or at least a 4 log reduction of yeasts, were observed in case the disinfectant comprises at least 1 part concentrate dissolved in 250 parts aqueous medium, e.g. water. In such case, it was found that the obtained disinfectant was still effective within 300 seconds.

The disinfectant may comprise:
- 0.1 - 2.5 weight-% hydrogen peroxide;
- 0.1 - 2.5 weight-% carboxylic acid and peroxy acid thereof; and
- 0.002 - 0.05 weight-% surfactant,
wherein the weight percentages are calculated based on the total weight of the disinfectant. Preferably, the amount of peroxy acid in the disinfectant is in the range of 0.0005 to 0.15 weight-% of the total weight of the disinfectant. Other preferred disinfectant compositions comprises up to 0.10 weight-% peroxy acid, up to 0.06 weight-% peroxy acid, up to 0.02 weight-% peroxy acid, up to 0.005 weight-% peroxy acid or up to 0.002 weight-% peroxy acid, wherein the weight percentages are calculated based on the total weight of the disinfectant.

In yet another aspect of the present invention, the present invention relates to a process for disinfecting a surface, comprising the steps of:
a) providing the concentrate of the present invention;
b) diluting the concentrate provided in step a) in a liquid medium, such as an aqueous medium;
c) applying the diluted concentrate of step b) onto a surface to be disinfected; and
d) allowing the applied diluted concentrate to interact with the surface to be disinfected.

Although it is not necessary to remove the remaining residue from the surface to be disinfected after the applied diluted concentrate is allowed to interact with the respective surface, since the residues are harmless, the above-described process may further comprises the step of e) removing remaining residue from the surface to be disinfected.

Effective disinfecting results are obtained wherein the time needed in step d) for allowing the applied diluted concentrate to interact with the surface to be disinfected does not exceed 300 seconds, i.e. the time needed in step d) for allowing the applied diluted concentrate to interact with the surface to be disinfected can be around 300 seconds or even lower. Preferably the time needed in step d) for allowing the applied diluted concentrate to interact with the surface to be disinfected does not exceed 120 seconds. More preferably the time needed for allowing the applied diluted concentrate to interact with the surface to be disinfected is in the range of 60 to 90 seconds.

In a further aspect, the present invention relates to the use of a surfactant selected from the group consisting of ionic surfactants in a concentrate for a disinfectant, wherein the concentrate comprises a hydrogen peroxide and at least one carboxylic acid and peroxy acid thereof, said at least one carboxylic acid is selected from the group consisting of alpha hydroxyl acids and wherein the weight ratio of carboxylic acid and peroxy acid to surfactant is about 50 : 1 to about 5000 : 1. Preferably, the weight ratio of carboxylic acid and peroxy acid to surfactant is about 50 : 1. In a preferred embodiment the weight ratio of hydrogen peroxide to carboxylic acid and peroxy acid is about 1 : 1.

As already stated above, the hydrotropic surfactant may comprise an anionic surfactant, such as a sulfosuccinate, e.g. an alkyl sulfosuccinate, in particular a dialkyl sulfosuccinate. Specifically preferred hydrotropic surfactants are selected form the group comprising an ammonium dinonyl sulfosuccinate, diamyl sodium sulfosuccinate, dicapryl sodium sulfosuccinate, diheptyl sodium sulfosuccinate, dihexyl sodium sulfosuccinate, diisobutyl sodium sulfosuccinate, ditridecyl sodium sulfosuccinate and/or dioctyl sodium sulfosuccinate.

The concentrate and/or disinfectant according to the present invention is particularly suitable for use in human hygiene, e.g. as a mouthwash or dental brace cleaning solution. The concentrate and/or disinfectant according to the present invention may further be used as a disinfectants and/or algaecides not intended for direct application to humans or animals, e.g. the disinfection of surfaces. In particular, the concentrate and/or disinfectant according to the present invention is particular suitable for use in the food and feed area, e.g. for the disinfection of equipment, containers, consumption utensils, surfaces or pipework associated with the production, transport, storage or consumption of food or feed (including drinking water) for humans and animals. Finally, the concentrate and/or disinfectant according to the present invention may be used for the cleaning of drinking water pipes.

### EXAMPLES

Efficacy tests were performed with one specific embodiment of the disinfectant according to the present invention (Example Composition B), as well as with a disinfectant of a traditional composition, i.e. without any surfactant (Example Composition A). Both compositions are shown in Table 1.

**Table 1. Concentrate compositions.**

| | Example Composition A | Example Composition B |
|---|---|---|
| Hydrogen peroxide | 25 % w/w | 25 % w/w |
| Lactic acid | 25 % w/w | 25 % w/w |
| Sodium dioctyl sulfosuccinate | | 0.5 % w/w |
| Water | Up to 100 % | Up to 100 % |

The Efficacy tests were performed according to the European standard suspension test EN-1276 as required for authorization of disinfectant products in EU member states (EN-1276 (2009) 'Chemical disinfectants and antiseptics - Quantitative suspension test for the evaluation of bactericidal activity of chemical disinfectants and antiseptics used in food, industrial, domestic and institutional areas - Test method and requirements (phase 2, step 1)').

The efficacy tests were performed with *Staphylococcus aureus* ATCC 6538. The initial concentration of *S. aureus* was 4.0 x 10⁶ cfu/g. Wherein the term 'cfu' refers to the colony-forming units.

With Example Composition A, the efficacy of 10x, 20x, 50x and 100x dilutions after a contact time of 5 minutes was investigated. With Composition B, the efficacy of 10x, 25x and 83x dilutions after contact times of 1, 2, and 5 minutes was investigated.

The tables 2 and 3 provide a summary of the results obtained in the EN-1276 suspension tests with Example Compositions A and B, expressed as ¹⁰Log (reduction), with 'reduction' being defined as cfu/g_initial / cfu/g_final.

**Table 2. Log reduction Example Composition A after 5 minutes.**

| Example Composition A - dilution | Log reduction *S. aureus* after 5 minutes |
|---|---|
| 10x | >5.6 |
| 20x | 4.4 |
| 50x | <1.1 |
| 100x | <0.1 |

**Table 3. Log reduction Example Composition B after 1, 2 and 5 minutes.**

| Example Composition B - dilution | Log reduction *S. aureus* after | | |
|---|---|---|---|
| | 1 minute | 2 minutes | 5 minutes |
| 10x | >5.6 | >5.6 | >5.6 |
| 25x | >5.6 | >5.6 | >5.6 |
| 83x | >5.6 | >5.6 | >5.6 |
| 250x | 4.35 | 4.73 | 5.09 |

As it can be derived from Tables 2 and 3, Example Composition A, without the surfactant, achieves the required 5 log units reduction in bacterial cell count (cfu's per unit) only at a 10x dilution after a contact time of 5 minutes, whereas Example Composition B achieves the required 5 log units reduction in bacterial cell count even at 83x dilution after a contact time of only 1 minute; at a low surfactant concentration of 0.006 % w/w. Even further, the Example Composition B achieves the required 5 log at a 250x dilution after a contact time of 5 minutes, i.e. a surfactant concentration of 0.002. Similar EN-1276 suspension tests were performed with *Pseudomonas aeruginosa, Escherichia coli, Enterococcus hirae* and *Candida albicans*. The efficacy of 10x, 25x, 80x and 250x dilutions of Example Composition B after contact times of 1, 2, and 5 minutes was investigated. The results are provided in Tables 4, 5, 6 and 7.

**Table 4. Log reduction Pseudomonas aeruginosa.**

| Example Composition B - dilution | 1 minute | 2 minutes | 5 minutes |
|---|---|---|---|
| 10x | >5.37 | >5.37 | >5.37 |
| 25x | >5.37 | >5.37 | >5.37 |
| 80x | >5.37 | >5.37 | >5.37 |
| 250x | 4.54 | 4.82 | 5.25 |

**Table 5. Log reduction Escherichia coli.**

| Example Composition B - dilution | 1 minute | 2 minutes | 5 minutes |
|---|---|---|---|
| 10x | >5.41 | >5.41 | >5.41 |
| 25x | >5.41 | >5.41 | >5.41 |
| 80x | >5.41 | >5.41 | >5.41 |
| 250x | 4.66 | 5.24 | >5.41 |

**Table 6. Log reduction Enterococcus hirae.**

| Example Composition B - dilution | 1 minute | 2 minutes | 5 minutes |
|---|---|---|---|
| 10x | >5.46 | >5.46 | >5.46 |
| 25x | >5.46 | >5.46 | >5.46 |
| 80x | >5.46 | >5.46 | >5.46 |
| 250x | 4.5 | 4.91 | 5.32 |

**Table 7. Log reduction Candida albicans.**

| Example Composition B - dilution | 1 minute | 2 minutes | 5 minutes |
|---|---|---|---|
| 10x | >4.49 | >4.49 | >4.49 |
| 25x | >4.49 | >4.49 | >4.49 |
| 80x | >4.49 | >4.49 | >4.49 |
| 250x | 3.12 | 3.12 | 3.79 |

## Claims

1. Concentrate for a disinfectant, comprising:
- hydrogen peroxide;
- at least one carboxylic acid and peroxy acid thereof, said at least one carboxylic acid is selected from the group consisting of alpha hydroxy acids; and
- at least one surfactant selected from the group consisting of ionic surfactants, **characterized in that** the weight ratio of carboxylic acid and peroxy acid to surfactant is 50 : 1 to 5000 : 1.

2. Concentrate according to claim 1, **characterized in that** the at least one surfactant comprises a sulfosuccinate, such as an alkyl sulfosuccinate, in particular a dialkyl sulfosuccinate.

3. Concentrate according to claim 1 or 2, **characterized in that** the at least one carboxylic acid is selected from lactic acid, citric acid, glycolic acid, 2-hydroxybutyric acid and combinations thereof.

4. Concentrate according to any of the preceding claims, comprising:
- 0.25 - 25 weight-% hydrogen peroxide;
- 0.25 - 25 weight-% carboxylic acid and peroxy acid thereof; and
- 0.005 - 0.5 weight-% surfactant,
wherein the weight percentages are calculated based on the total weight of the concentrate.

5. Concentrate according to any of the preceding claims, **characterized in that** the concentrate comprises up to 1.5 weight-% peroxy acid, wherein the weight percentage is calculated based on the total weight of the concentrate.

6. Concentrate according to any of the preceding claims, **characterized in that** the pH of the concentrate is in the range of 2 to 5.

7. Disinfectant comprising the concentrate according to any of the preceding claims, **characterized in that** the concentrate is solubilized in a liquid medium, such as an aqueous medium.

8. Disinfectant according to claim 7, **characterized in that** the pH of the disinfectant is in the range of 2 to 5.

9. Disinfectant according to claim 7 or 8, **characterized in that** the disinfectant comprises at least 1 part concentrate dissolved in 250 parts aqueous medium.

10. Disinfectant according to claim 9, **characterized in that** the amount of peroxy acid is in the range of 0.0005 to 0.15 weight-% of the total weight of the disinfectant.

11. Process for disinfecting a surface, comprising the steps of:
a) providing the concentrate according to any of claims 1-6;
b) diluting the concentrate provided in step a) in a liquid medium, such as an aqueous medium;
c) applying the diluted concentrate of step b) onto a surface to be disinfected;
d) allowing the applied diluted concentrate to interact with the surface to be disinfected; and, optionally,
e) removing remaining residue from the surface to be disinfected.

12. Process according to claim 11, **characterized in that** the time needed in step d) for allowing the applied diluted concentrate to interact with the surface to be disinfected does not exceed 300 seconds, preferably the time needed is in the range of 60 to 90 seconds.

13. Use of a surfactant selected from the group consisting of ionic surfactants in a concentrate for a disinfectant, wherein the concentrate comprises a hydrogen peroxide and at least one carboxylic acid and peroxy acid thereof, said at least one carboxylic acid is selected from the group consisting of alpha hydroxyl acids, **characterized in that** the weight ratio of carboxylic acid and peroxy acid to surfactant is 50 : 1 to 5000 : 1.

14. Use according to claim 13, **characterized in that** the surfactant comprises a sulfosuccinate, such as an alkyl sulfosuccinate, in particular a dialkyl sulfosuccinate.

15. Use of the concentrate according to any of claims 1-6 and/or disinfectant according to any of claims 7-10 for human hygiene, as a disinfectants and/or algaecides not intended for direct application to humans or animals, for the food and feed area or for the cleaning of drinking water pipes.
